# EUROPÄISCHE PATENTSCHRIFT

(11) **EP 1 480 686 B1**
(45) Veröffentlichungstag und Bekanntmachung des Hinweises auf die Patenterteilung: **12.09.2007**
(21) Anmeldenummer: 03720314.8
(22) Anmeldetag: 01.03.2003
(51) Int. Cl.: A61L 2/26

(54) **STERILBEHÄLTER UND FILTEREINHEIT FÜR EINEN STERILBEHÄLTER**
STERILE CONTAINER AND FILTER UNIT FOR A STERILE CONTAINER
CONTENEUR STERILE ET ENSEMBLE DE FILTRATION DESTINE A UN CONTENEUR STERILE

(30) Priorität: 06.03.2002 DE 20203984 U; 27.08.2002 DE 10240597
(43) Veröffentlichungstag der Anmeldung: 01.12.2004
(73) Patentinhaber: Aesculap AG & Co. KG, 78532 Tuttlingen (DE)
(72) Erfinder: JAKAB, Mariana, 78532 Tuttlingen (DE); OERTMANN, Friedrich-Wilhelm, 78532 Tuttlingen (DE); RENNER, Torsten, 07607 Eisenberg (DE); SCHUSTER, Stefan, 78058 Villingen-Schwenningen (DE); SCHWANKE, Wolfgang, 78604 Rietheim-Weilheim (DE)
(74) Vertreter: Hoeger, Stellrecht & Partner Patentanwälte
(86) Internationale Anmeldenummer: PCT/EP2003/002134
(87) Internationale Veröffentlichungsnummer: WO 2003/074094

(56) Entgegenhaltungen:
- DE-A- 19 753 671
- DE-U- 29 812 996
- US-A- 2 071 881
- US-A- 5 736 043

## Beschreibung

Die Erfindung betrifft eine Filtereinheit für einen Sterilbehälter, insbesondere zur Aufnahme und sterilen Aufbewahrung von chirurgischem Besteck oder Material, umfassend einen Aufnahmebehälter, einen Deckel zum Verschließen des Aufnahmebehälters und eine von der Filtereinheit verschließbare Gasaustauschöffnung, wobei die Filtereinheit ein von mindestens einem Trägerelement gehaltenes Sterilfilter umfaßt, wobei die Filtereinheit mindestens einen von dem Sterilfilter getrennten Griffbereich zum Greifen und Halten der Filtereinheit aufweist und wobei der Griffbereich eine Gifffläche umfaßt, wobei das mindestens eine Trägerelement einen ersten, äußeren Halterahmen umfaßt, wobei das Sterilfilter an dem äußeren Halterahmen gehalten ist, wobei das mindestens eine Trägerelement eine zweiten, inneren Halterahmen umfaßt und wobei der innere Halterahmen innerhalb des ersten, äußeren Halterahmens angeordnet und mittels Verbindungselementen mit diesem verbunden ist.

Ferner betrifft die vorliegende Erfindung einen Sterilbehälter, insbesondere zur Aufnahme und sterilen Aufbewahrung von chirurgischem Besteck oder Material, umfassend einen durch einen Behälterboden und Behälterwände gebildeten Aufnahmeraum, einen Deckel zum Verschließen des Aufnahmeraums und eine von einer Filtereinheit verschließbare Gasaustauschöffnung.

In derartigen Sterilbehältern wird beispielsweise chirurgisches Besteck oder Material sterilisiert und gelagert. Damit während des Sterilisiervorgangs Heißdampf in den Sterilbehälter hineingelangen kann, wird eine Gasaustauschöffnung benötigt. Um jedoch zu verhindern, daß nach dem Sterilisieren Keime, Bakterien oder dergleichen in den Sterilbehälter hineingelangen können, wird die Gasaustauschöffnung bereits vor dem Sterilisiervorgang mit einem Sterilfilter verschlossen, welches einen Gasaustausch zuläßt, jedoch keine unerwünschten Erreger in den Sterilbehälter eindringen läßt.

Beim Austauschen der Filtereinheiten am Sterilbehälter erweist es sich als nachteilig, daß bei einer nicht sehr sorgfältigen Vorgehensweise beim Austauschen das Sterilfilter mit Fingern in Kontakt kommen und dadurch verschmutzt werden kann.

Aus der US 5,736,043 sind Filtereinheiten bekannt, die radial nach außen abstehende, an einem Trägerelement angeordnete Griffbereiche aufweisen.

Daher ist es Aufgabe der vorliegenden Erfindung, eine Filtereinheit und einen Sterilbehälter der eingangs beschriebenen Art so zu verbessern, daß die Stabilität der Filtereinheit erhöht wird.

Diese Aufgabe wird bei einer Filtereinheit der eingangs beschriebenen Art erfindungsgemäß dadurch gelöst, daß das Sterilfilter zwischen zwei der genamten Trägerelemente angeordnet ist und daß die Verbindungselemente der beiden, miteinander verbundenen Trägerelemente um eine parallel zu einer Normalenrichtung zu einer von dem Sterilfilter definierten Ebene orientierte Drehachse relativ zueinander verdreht angeordnet sind.

Durch den von dem Sterilfilter getrennten Griffbereich ist es möglich, daß eine Person beim Austausch die Filtereinheit an dem speziell vorgesehenen Griffbereich erfassen und halten kann. Am Griffbereich läßt sich so die Filtereinheit sicher erfassen, wodurch die Gefahr vermindert wird, daß die Person mit ihren Fingern auf das Sterilfilter abrutscht und dieses verschmutzt. Damit eine möglichst große Durchtrittsfläche durch das Sterilfilter gebildet wird, gleichzeitig eine sichere und stabile Halterung des Sterilfilters gewährleistet ist, ist vorgesehen, daß das mindestens eine Trägerelement einen ersten, äußeren Halterahmen umfaßt und daß das Sterilfilter an dem äußeren Halterahmen gehalten ist. Günstig ist es, daß das mindestens eine Trägerelement einen zweiten, inneren Halterahmen umfaßt und daß der innere Halterahmen innerhalb des ersten, äußeren Halterahmens angeordnet und mittels Verbindungselementen mit diesem verbunden ist. Besonders bei hochflexiblen, dünnen oder besonders empfindlichen Sterilfiltern wird durch diese besondere Ausgestaltung das Sterilfilter zusätzlich stabilisiert. Vorteilhaft ist es ferner aber auch, daß die Verbindungselemente der beiden, miteinander verbundenen Trägerelemente um eine parallel zu einer Normalenrichtung zu einer von dem Sterilfilter definierten Ebene orientierte Drehachse relativ zueinander verdreht angeordnet sind. Zwar wird dadurch die Durchtrittsfläche durch das Sterilfilter etwas verringert, die Stabilität der Filtereinheit jedoch erhöht, da bei einem versehentlichen Abrutschen eines Fingers beim Halten der Filtereinheit das Sterilfilter nicht so einfach durchgedrückt werden kann. Die Verbindungselemente bilden für Finger unbeschädigbare Barrieren.

Vorzugsweise ist der mindestens eine Griffbereich an dem mindestens einen Trägerelement angeordnet. Dadurch wird die Stabilität der Filtereinheit erhöht, insbesondere können Trägerelement und Griffbereich einstückig ausgebildet werden.

Günstig ist es, wenn der mindestens eine Griffbereich eine an dem mindestens einen Trägerelement angeordnete Ausnehmung umfaßt. Ergreift eine Bedienperson die Filtereinheit an dem Griffbereich, so werden die Finger der Bedienperson in der Ausnehmung gehalten, ein Abrutschen wird verhindert.

Bei einer bevorzugten Ausführungsform der Erfindung können mehrere, an dem mindestens einen Trägerelement angeordnete Griffbereiche vorgesehen sein. Dadurch wird die Wahrscheinlichkeit erhöht, daß die Filtereinheit bei einem zufälligen Ergreifen an einem der Griffbereiche erfaßt wird. Darüber hinaus ermöglichen es mehrere Griffbereiche, daß die Filtereinheit beidseitig, beispielsweise mit zwei Händen erfaßt werden kann, wodurch der Austausch der Filtereinheit erleichtert wird.

Vorteilhaft ist es, wenn das Sterilfilter zwischen zwei Trägerelementen klemmend gehalten ist. Auf diese Weise wird es beidseitig zumindest teilweise gegen einen Fingerkontakt geschützt.

Günstig ist es, wenn das Sterilfilter mit dem mindestens einen Trägerelement unlösbar verbunden ist, insbesondere verklebt oder verschweißt. Dadurch wird verhindert, daß das Sterilfilter herausfallen kann. Außerdem wird der Austausch des Sterilfilters erleichtert, da es als Einheit mit dem mindestens einen Trägerelement ausgetauscht wird. Ferner wird das Sterilfilter durch das mindestens eine Trägerelement stabilisiert, was insbesondere bei üblicherweise verwendeten, flexiblen Sterilfiltern vorteilhaft ist.

Um das Sterilfilter noch sicherer zu schützen und die Stabilität zusätzlich zu erhöhen, können die zwei Trägerelemente unlösbar miteinander verbunden sein, insbesondere verklebt oder verschweißt.

Eine besonders große Durchtrittsfläche für einen Gasaustausch ergibt sich, wenn die inneren und/oder äußeren Halterahmen ringförmig ausgebildet sind.

Besonders einfach und kostengünstig ist die Herstellung einer Filtereinheit, wenn die Verbindungselemente radiale, von einem Zentrum der inneren und äußeren Halterahmen weg gerichtete Stege umfassen.

Noch sicherer wird das Sterilfilter gehalten, wenn die Verbindungselemente der beiden, miteinander verbundenen Trägerelemente in einer Normalenrichtung zu einer von dem Sterilfilter definierten Ebene überdeckend angeordnet sind. Dadurch wird bei beidseitiger Halterung des Sterilfilters zwischen beiden Trägerelementen im Bereich der Verbindungselemente eine besonders stabile Halterung des Sterilfilters erreicht. Ferner wird die Durchtrittsfläche des Sterilfilters für einen Gasaustausch maximiert.

Günstig ist es, wenn die relativ zueinander verdreht angeordneten Verbindungselemente der beiden, miteinander verbundenen Trägerelemente relativ zueinander um einen Drehwinkel versetzt sind, der sich aus dem Quotient zwischen 360° und der doppelten Anzahl der Verbindungselemente ergibt. Dadurch dienen insgesamt alle Verbindungselemente der Filtereinheit als Stabilisierung für das Sterilfilter, wohingegen bei einer überdeckenden Anordnung der Verbindungselemente nur die einfache Zahl der Verbindungselemente zur Stabilisierung der Filtereinheit beiträgt.

Damit die Filtereinheit besonders sicher erfaßt werden kann, ist es günstig, wenn der mindestens eine Griffbereich an dem äußeren und/oder dem inneren Halterahmen angeordnet ist.

Um ein unbeabsichtigtes Abrutschen von Fingern auf das Sterilfilter zu verhindern, kann es von Vorteil sein, wenn der äußere und/oder der innere Halterahmen eine Rahmenbreite in radialer Richtung aufweisen und wenn der mindestens eine Griffbereich eine Griffbereichsbreite in radialer Richtung aufweist, die mindestens abschnittsweise größer ist als die Rahmenbreite von an den Griffbereich angrenzenden Abschnitten des äußeren und/oder des inneren Halterahmens. Der demnach gegenüber angrenzenden Abschnitten der Halterahmen verbreiterte Griffbereich ermöglicht ein sicheres Erfassen der Filtereinheit, wodurch die Gefahr eines Verschmutzens oder Beschädigens des Sterilfilters vermindert wird.

Um ein Abrutschen von Fingern vom Griffbereich zusätzlich zu erschweren, kann der mindestens eine Griffbereich ein Fingerrückhalteelement umfassen. Das Fingerrückhalteelement hält Finger zurück, welche vom Griffbereich abgleiten könnten. Das Fingerrückhalteelement bildet somit in beliebiger Weise eine Barriere für einen Finger.

Vorzugsweise umfaßt das Fingerrückhalteelement einen die Grifffläche des Griffbereichs umgebenden Rand. Ein die Grifffläche des Griffbereichs erfassender Finger wird durch den zumindest abschnittsweise die Grifffläche umgebenden Rand davon abgehalten, von der Grifffläche abzurutschen.

Einen besonders guten Halt kann eine strukturierte und/oder aufgerauhte Grifffläche bieten.

Die eingangs gestellte Aufgabe wird bei einem Steril behälter der eingangs beschriebenen Art erfindungsgemäß dadurch gelöst, daß die Filtereinheit eine der oben beschriebenen, erfindungsgemäßen Filtereinheiten umfaßt.

Indem der Sterilbehälter mit einer derartigen Filtereinheit versehen wird, wird die Gefahr eines Verschmutzens oder Beschädigens der Filtereinheit verringert, so daß insgesamt die Funktionalität des Sterilbehälters verbessert wird. Die Gefahr des Eindringens von Keimen oder Erregern in das Innere des Sterilbehälters nach dem Sterilisieren wird vermindert.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht eines Sterilbehälters;
- Figur 2:: eine ausschnittsweise Querschnittsansicht durch einen Deckel des In Figur 1 dargestellten Sterilbehälters längs Linie 2-2;
- Figur 3:: eine perspektivische Ansicht einer ersten Ausführungsform einer Filtereinheit; und
- Figur 4:: eine perspektivische Ansicht einer Ausführungsform einer Filtereinheit, welche nicht teil der vorliegenden Erfindung ist.

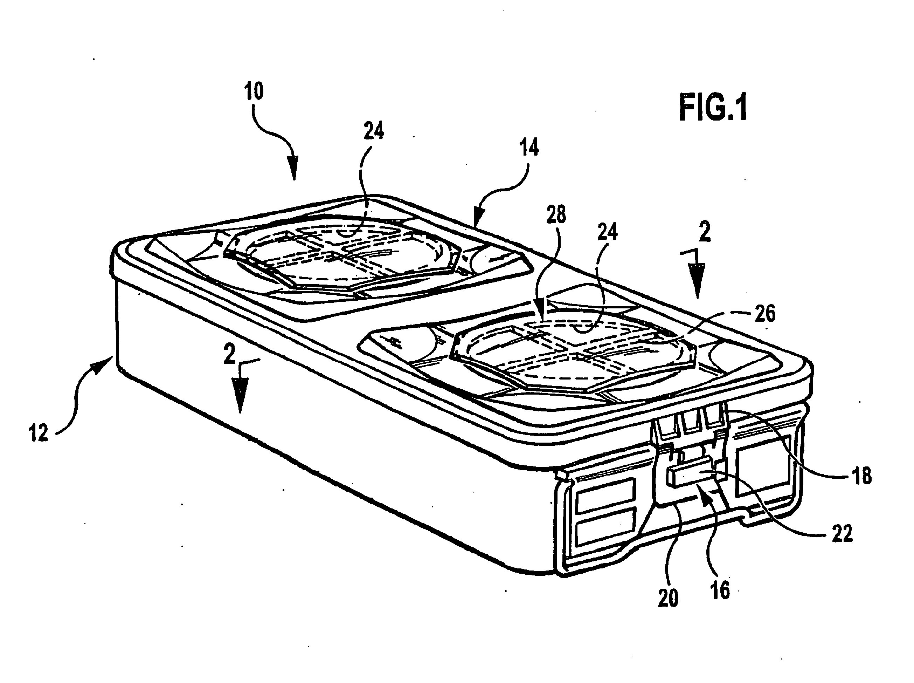

In Figur 1 ist ein insgesamt mit dem Bezugszeichen 10 versehener Sterilbehälter dargestellt, mit einer Wanne 12 zum Aufnehmen beispielsweise von chirurgischem Besteck oder Material und mit einem die Wanne 12 verschließenden Deckel 14.

Der Deckel 14 ist über zwei jeweils stirnseitig angeordnete Verschlüsse 16 mit der Wanne 12 verbindbar. Jeder Verschluß umfaßt einen stirnseitig am Deckel 14 angeordneten Lagerbock 18 und einen schwenkbar daran gelagerten Bügel 20, welcher in einem verriegelten, Zustand, wie er In Figur 1 dargestellt ist, einen an der Wanne 12 angeordneten Verriegelungsvorsprung 22 umgreift.

Um einen Gasaustausch zwischen einer Umgebung und dem Inneren des Sterilbehälters 10 zu ermöglichen, sind im Deckel symmetrisch zwei im wesentlichen kreisrunde Belüftungsöffnungen 24 angeordnet, welche von zwei sich kreuzenden Stegen 26 überspannt werden.

Auf einer Außenseite des Deckels 14 wird jede der beiden Belüftungsöffnungen 24 von einem Schutzdeckel 28 vollständig überdeckt.

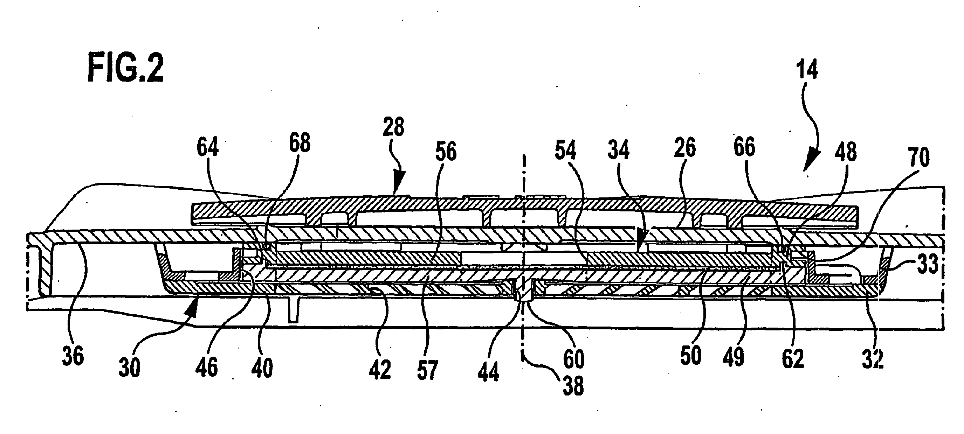

In der Schnittansicht in Figur 2 ist eine die Belüftungsöffnung 24 im Innern des Deckels 14 vollständig überdeckende Filterkassette 30 dargestellt, welche ein halbschaliges Filtergehäuse 32 mit einer darin gelagerten Filtereinheit 34 umfaßt. Das Filtergehäuse 32 umfaßt einen ringförmigen, in Richtung auf den Deckel 14 hin federnd vorgespannt gehaltenen Flltergehäuserahmen 33 zur Aufnahme der Filtereinheit 34. Der Filtergehäuserahmen 33 wird auf einer einer Deckelunterseite 36 abgewandten Seite mit einem Boden 40 bedeckt, so daß durch die beiden Teile eine flache, zylindrische, in Richtung auf die Deckelunterseite 36 hin geöffnete Filteraufnahme 46 gebildet wird. Die Filterkassette 30 ist Im wesentlichen bezogen auf eine zum Deckel 14 senkrecht orientierte Symmetrieachse symmetrisch ausgebildet. Der Boden 40 Ist mit schräg zum Boden 40 geneigten, Durchgangsöffnungen bildenden Längsschlitzen 42 und einer zentralen Bohrung 44 versehen.

Die Filtereinheit 34 umfaßt zwei Klemmscheiben, nämlich eine der Deckelunterseite 36 zugewandte obere Klemmscheibe 48 und eine untere Klemmscheibe 49, zwischen denen ein scheibenförmiges, flaches Sterilfilter gehalten ist, beispielsweise ein als Dauerfilter einsetzbares PTFE-Filter.

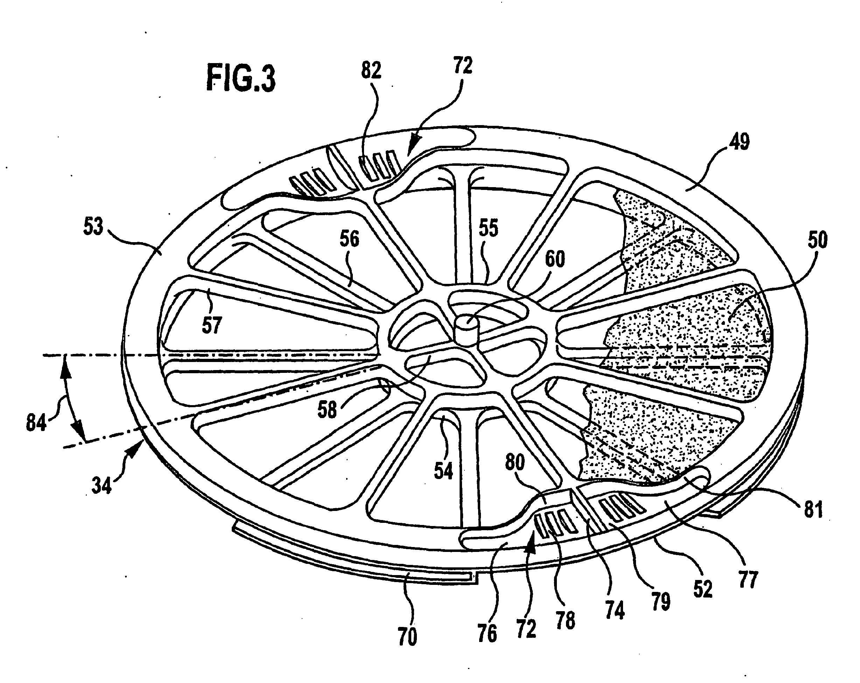

Wie in den Figuren 2 und 3 zu erkennen, umfaßt jede der beiden Klemmscheiben 48 und 49 einen äußeren Klemmring 52 bzw. 53 und einen inneren Klemmring 54 bzw. 55, welche über jeweils acht radial verlaufende Speichen 56 bzw. 57 miteinander verbunden sind. Zusätzlich ist im Innern des inneren Klemmrings 55 der unteren Klemmscheibe 49 zu Stabilisierungszwecken eine Steganordnung 58 aus zwei sich kreuzenden Stegen angebracht, die zentral einen von der Deckelunterseite 36 weg weisenden zylindrischen Zentrierzapfen 60 trägt.

Zur Verbindung der unteren Klemmscheibe 49 mit der oberen Klemmscheibe 48 ist die obere Klemmscheibe 48 mit einer Ringnut 62 versehen, in die ein in Richtung auf die Deckelunterseite 36 weisender Ringvorsprung 64 eingreift. In eine weitere, in Richtung auf die Deckelunterseite 36 hin geöffnete Ringnut der oberen Klemmscheibe ist ein Dichtring 68 eingesetzt, mit dem die obere Klemmscheibe 48 und damit die gesamte Filterkassette 30 gegenüber der Deckelunterseite 36 abgedichtet werden.

Um die Filtereinheit 34 mit dem Filtergehäuse 32 zu verbinden, ist eine Bajonettverbindung zwischen dem Filtergehäuserahmen 33 und der oberen Klemmscheibe vorgesehen. Hierzu trägt die obere Klemmscheibe 48 mehrere, über ihren Umfang radial nach außen geöffnete Bajonettnute 70, in welche ein korrespondierender, am Filtergehäuserahmen 33 radial nach innen gerichteter Vorsprung eintauchen kann.

Ferner sind an der Filtereinheit 34 zwei diametral einander gegenüberliegende Griffbereiche 72 ausgebildet, und zwar am äußeren Klemmring 53 der unteren Klemmscheibe 49. Jeder Griffbereich 72 ist sich symmetrisch an eine Speiche 57 anschließend symmetrisch zu dieser ausgebildet, wobei eine Verlängerung 74 der Speiche 57 zwei Vertiefungen 76 und 77 voneinander trennt, welche in ihrem Boden jeweils eine Grifffläche 78 bzw. 79 und einen in Form eines Vorsprungs von der jeweiligen Grifffläche 78 bzw. 79 abstehenden Rand 80 bzw. 81 tragen. Jede der beiden Griffflächen 78 und 79 ist zusätzlich mit einer Rippenstruktur 82 versehen. Anstelle der Rippenstruktur 82 kann die Grifffläche 78 bzw. 79 auch in beliebig anderer Form aufgerauht oder strukturiert sein.

Die Vertiefungen 76 und 77 weisen an Ihrem von der Verlängerung 74 weg weisenden Ende eine Breite auf, die wenig kleiner ist als die Breite des äußeren Klemmrings 53 in radialer Richtung. In Richtung auf die Verlängerung 74 hin verbreitert sich die Grifffläche 78 bzw. 79 einseitig in Richtung auf die Symmetrieachse 38 hin. Sie ist zumindest auf ihrer in Richtung auf die Symmetrieachse 38 hin weisenden Seite von dem Rand 80 begrenzt, kann jedoch auch vollständig von dem Rand 80 umgeben sein. Der Rand 80 dient insbesondere als Fingerrückhalteelement, um in die Vertiefungen 76 bzw. 77 eintauchenden Finger an einem Abrutschen auf das Sterilfilter 50 hin zu hindern.

Die Fliterkassette 30 wird zusammengesetzt, indem die Filtereinheit 34 an den Griffbereichen 72 mit Fingern ergriffen und In den bereits am Deckel 14 montierten Filtergehäuserahmen 33 in Richtung der Symmetrieachse 38 eingesetzt und anschließend um diese verdreht wird. Durch die Bajonettverbindung wird die Filtereinheit 34 an dem Filtergehäuserahmen 33 gehalten. Schließlich wird der Boden 40 mit seiner Bohrung 44 über den Zentrierzapfen 60 geschoben und mit weiteren Verriegelungsvorsprüngen im Bereich aneinander anliegender Flächen zwischen dem Boden 40 und dem Flltergehäuserahmen 33 verbunden.

Bei dem in Figur 3 dargestellten Ausführungsbeispiel einer Filtereinheit 34 sind die beiden Klemmscheiben 48 und 49 so miteinander verbunden, daß die Speichen 56 und 57 relativ zueinander versetzt um einen Drehwinkel 84 von 22,5° verlaufen. Der Sterilfilter 50 wird somit nicht zwischen zwei Speichen 56 und 57 geklemmt, sondern liegt jeweils wechselseitig auf einer der beiden Speichen 56 bzw. 57 auf. Durch diese relative Ausrichtung der Speichen 56 und 57 zueinander wird der Sterilfilter zusätzlich gegen eine Beschädigung geschützt, denn im Abstand von 22,5° schützt jeweils eine der Speichen 56 oder 57 das Sterilfilter gegen Durchdrücken, falls eine Person oder ein Gegenstand auf die Filtereinheit 34 greifen oder gelangen sollte.

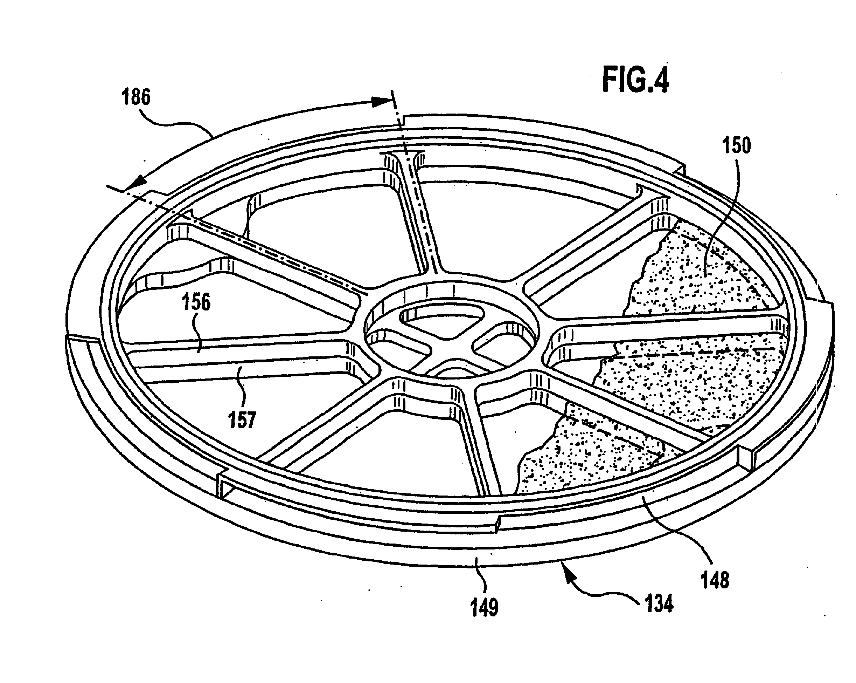

In Figur 4 Ist ein insgesamt mit dem Bezugszeichen 134 versehenes Ausführungsbeispiel einer Filtereinheit dargestellt welche nicht Teil der vorliegenden Erfindung ist und bei der identische Teile mit denselben Bezugsziffern und zusätzlich vorangestellter 1 versehen sind. Die Filtereinheiten 34 und 134 unterscheiden sich dadurch, daß bei der Filtereinheit 134 die Speichen 156 und 157 einander überdeckend angeordnet sind, so daß ein Abstand der Speichen 156 und 157 einem Speichenwinkel 186 von 45° entspricht. Der Sterilfilter 150 wird demnach zwischen zwei Speichen 156 und 157 zusätzlich geklemmt, allerdings ist dabei die freie Fläche zwischen benachbarten Speichen 156 doppelt so groß wie bei der Filtereinheit 34.

## Patentansprüche

1. Filtereinheit für einen Sterilbehälter, insbesondere zur Aufnahme und sterilen Aufbewahrung von chirurgischem Besteck oder Material, umfassend einen Aufnahmebehälter, einen Deckel zum Verschließen des Aufnahmebehälters und eine von der Filtereinheit verschließbare Gasaustauschöffnung, wobei die Filtereinheit ein von mindestens einem Trägerelement gehaltenes Sterilfilter umfaßt, wobei die Filtereinheit (34) mindestens einen von dem Sterilfilter (50) getrennten Griffbereich (72) zum Greifen und Halten der Filtereinheit (34) aufweist und daß der Griffbereich (72) eine Grifffläche (78) umfaßt, wobei das mindestens eine Trägerelement (48, 49) einen ersten, äußeren Halterahmen (52, 53) umfaßt, wobei das Sterilfilter (50) an dem äußeren Halterahmen (52, 53) gehalten ist, wobei das mindestens eine Trägerelement (48, 49) einen zweiten, inneren Halterahmen (54, 55) umfaßt und wobei der innere Halterahmen (54, 55) innerhalb des ersten, äußeren Halterahmens (52, 53) angeordnet und mittels Verbindungselementen (56, 57) mit diesem verbunden ist, **dadurch gekennzeichnet, dass** das Sterilfilter zwischen zwei der genamten Trägerelemente angeordnet ist und dass die Verbindungselemente (56, 57) der beiden, miteinander verbundenen Trägerelemente (48, 49) um eine parallel; zu einer Normalenrichtung zu einer von dem Sterilfilter (50) definierten Ebene orientierte Drehachse (38) relativ zueinander verdreht angeordnet sind.

2. Filtereinheit nach Anspruch 1, **dadurch gekennzeichnet, daß** der mindestens eine Griffbereich (72) an mindestens einem Trägerelement (49) angeordnet ist.

3. Filtereinheit nach einem der Ansprüche 1 oder 2, **dadurch gekennzeichnet, daß** der mindestens eine Griffbereich (72) eine an mindestens einen Trägerelement (49) angeordnete Ausnehmung (76, 77) umfaßt.

4. Filtereinheit nach einem der voranstehenden Ansprüche, **dadurch gekennzeichnet, daß** mehrere, an mindestens einem Trägerelement (49) angeordnete Griffbereiche (72) vorgesehen sind.

5. Filtereinheit nach einem der voranstehenden Ansprüche, **dadurch gekennzeichnet, daß** das Sterilfilter (50) zwischen den Trägerelementen (48, 49) klemmend gehalten ist.

6. Filtereinheit nach einem der voranstehenden Ansprüche, **dadurch gekennzeichnet, daß** das Sterilfilter (50) mit mindestens einem Trägerelement (48, 49) unlösbar verbunden ist, insbesondere verklebt oder verschweißt.

7. Filtereinheit nach einem der Ansprüche 5 oder 6, **dadurch gekennzeichnet, daß** die beiden Trägerelemente (48, 49) unlösbar miteinander verbunden sind, insbesondere verklebt oder verschweißt.

8. Filtereinheit nach einem der voranstehenden Ansprüche, **dadurch gekennzeichnet, daß** die inneren und/oder äußeren Halterahmen (52, 53; 54, 55) ringförmig ausgebildet sind.

9. Filtereinheit nach einem der voranstehenden Ansprüche, **dadurch gekennzeichnet, daß** die Verbindungselemente radiale, von einem Zentrum der inneren und äußeren Halterahmen (52, 53; 54, 55), weg geriditete Stege (56, 57) umfassen.

10. Filtereinheit nach einem der voranstehenden Ansprüche, **dadurch gekennzeichnet, daß** Verbindungselemente (156, 157) der beiden, miteinander verbundenen Trägerelemente (148, 149) in einer Normalenrichtung (138) zu einer von dem Sterilfilter (150) definierten Ebene überdeckend angeordnet sind.

11. Filtereinheit nach einem der voranstehenden Ansprüche, **dadurch gekennzeichnet, daß** die relativ zueinander verdreht angeordneten Verbindungselemente (56, 57) der beiden, miteinander verbundenen Trägerelemente (48, 49) relativ zueinander um einen Drehwinkel (84) versetzt sind, der sich aus dem Quotient zwischen 360° und der doppelten Anzahl der Verbindungselemente (56; 57) ergibt.

12. Filtereinheit nach einem der voranstehenden Ansprüche, **dadurch gekennzeichnet, daß** der mindestens eine Griffbereich (72) an einem der äußeren und/oder dem inneren Halterahmen (52, 53; 54, 55) angeordnet ist.

13. Filtereinheit nach einem der voranstehenden Ansprüche, **dadurch gekennzeichnet, daß** der äußere und/oder der innere Halterahmen (52, 53; 54, 55) eines Trägerelements eine Rahmenbreite in radialer Richtung aufweisen und daß mindestens eine Griffbereich (72) eine Griffbereichsbreite in radialer Richtung aufweist, die mindestens abschnittsweise größer ist als die Rahmenbreite von an den Griffbereich (72) angrenzenden Abschnitten des äußeren und/oder des inneren Halterahmens (52, 53; 54, 55).

14. Filtereinheit nach einem der voranstehenden Ansprüche, **dadurch gekennzeichnet, daß** der mindestens eine Griffbereich (72) ein Fingerrückhalteelement (80, 81) umfaßt.

15. Filtereinheit nach Anspruch 14, **dadurch gekennzeichnet, daß** das Fingerrückhalteelement einen die Grifffläche (78, 79) des Griffbereichs (72) umgebenden Rand (80, 81) umfaßt.

16. Filtereinheit nach einem der voranstehenden Ansprüche, **dadurch gekennzeichnet, daß** die Grifffläche (78, 79) strukturiert und/oder aufgerauht ist.

17. Sterilbehälter, insbesondere zur Aufnahme und sterilen Aufbewahrung von chirurgischem Besteck oder Material, umfassend einen durch einen Behälterboden und Behälterwände gebildeten Aufnahmeraum, einen Deckel zum Verschließen des Aufnahmeraums und eine von einer Filtereinheit verschließbare Gasaustauschöffnung, **dadurch gekennzeichnet, daß** die Filtereinheit eine Filtereinheit (34; 134) nach einem der voranstehenden Ansprüche umfaßt.

## Claims

1. Filter unit for a sterile container, in particular, for accommodating and storing in a sterile manner surgical instruments or material, comprising a receptacle, a lid for closing the receptacle, and a gas exchange opening closable by the filter unit, the filter unit comprising a sterile filter held by at least one carrier element, the filter unit (34) comprising at least one grip area (72) separate from the sterile filter (50) for gripping and holding the filter unit (34), and the grip area (72) comprising a grip surface (78), the at least one carrier element (48, 49) comprising a first, outer holding frame (52, 53), the sterile filter (50) being held on the outer holding frame (52, 53), the at least one carrier element (48, 49) comprising a second, inner holding frame (54, 55), and the inner holding frame (54, 55) being arranged within the first, outer holding frame (52, 53) and being connected thereto by connecting elements (56, 57), **characterized in that** the sterile filter is arranged between two of the said carrier elements, and that the connecting elements (56, 57) of the two carrier elements (48, 49) joined to each other are arranged such that they are rotated relative to one another about an axis of rotation (38) orientated parallel to a direction perpendicular to a plane defined by the sterile filter (50).

2. Filter unit in accordance with claim 1, **characterized in that** the at least one grip area (72) is arranged on at least one carrier element (49).

3. Filter unit in accordance with claim 1 or 2, **characterized in that** the at least one grip area (72) comprises a recess (76, 77) arranged on at least one carrier element (49).

4. Filter unit in accordance with any one of the preceding claims, **characterized in that** several grip areas (72) arranged on at least one carrier element (49) are provided.

5. Filter unit in accordance with any one of the preceding claims, **characterized in that** the sterile filter (50) is held in a clamped manner between the carrier elements (48, 49).

6. Filter unit in accordance with any one of the preceding claims, **characterized in that** the sterile filter (50) is undetachably connected to at least one carrier element (48, 49), in particular, adhesively joined or welded.

7. Filter unit in accordance with claim 5 or 6, **characterized in that** the two carrier elements (48, 49) are undetachably connected to each other, in particular, adhesively joined or welded.

8. Filter unit in accordance with any one of the preceding claims, **characterized in that** the inner and/or outer holding frames (52, 53; 54, 55) are of ring-shaped construction.

9. Filter unit in accordance with any one of the preceding claims, **characterized in that** the connecting elements comprise radial spokes (56, 57) orientated away from a center of the inner and outer holding frames (52, 53; 54, 55).

10. Filter unit in accordance with any one of the preceding claims, **characterized in that** the connecting elements (156, 157) of the two carrier elements (148, 149) joined to each other are arranged so as to overlap one another in a direction (138) perpendicular to a plane defined by the sterile filter (150).

11. Filter unit in accordance with any one of the preceding claims, **characterized in that** the connecting elements (56, 57), which are arranged so as to be rotated relative to one another, of the two carrier elements (48, 49) which are joined to each other, are offset relative to one another through an angle of rotation (84) resulting from the quotient between 360° and twice the number of the connecting elements (56; 57).

12. Filter unit in accordance with any one of the preceding claims, **characterized in that** the at least one grip area (72) is arranged on one of the outer and/or the inner holding frames (52, 53; 54, 55).

13. Filter unit in accordance with any one of the preceding claims, **characterized in that** the outer and/or the inner holding frame (52, 53; 54, 55) of one of the carrier elements have a frame width in radial direction, and that the at least one grip area (72) has a grip area width in radial direction, which is larger at least in sections thereof than the frame width of sections of the outer and/or the inner holding frame (52, 53; 54, 55) adjoining the grip area (72).

14. Filter unit in accordance with any one of the preceding claims, **characterized in that** the at least one grip area (72) comprises a finger retaining element (80, 81).

15. Filter unit in accordance with claim 14, **characterized in that** the finger retaining element comprises a rim (80, 81) surrounding the grip surface (78, 79) of the grip area (72).

16. Filter unit in accordance with any one of the preceding claims, **characterized in that** the grip surface (78, 79) is structured and/or roughened.

17. Sterile container, in particular, for accommodating and storing in a sterile manner surgical instruments or material, comprising an accommodating space formed by a container bottom and container walls, a lid for closing the accommodating space, and a gas exchange opening closable by a filter unit, **characterized in that** the filter unit comprises a filter unit (34; 134) in accordance with any one of the preceding claims.

## Revendications

1. Unité de filtrage pour un conteneur stérile, en particulier destiné à recevoir et à conserver de manière stérile des ustensiles ou matériels chirurgicaux, comportant un récipient, un couvercle pour fermer le récipient et une ouverture d'échange de gaz pouvant être fermée par l'unité de filtrage, l'unité de filtrage comportant un filtre stérile maintenu par au moins un élément support, l'unité de filtrage (34) comportant au moins une zone de préhension (72), séparée du filtre stérile (50), pour saisir et maintenir l'unité de filtrage (34), et la zone de préhension (72) comportant une surface de préhension (78), ledit au moins un élément support (48, 49) comportant un premier cadre de fixation (52, 53) extérieur, le filtre stérile (50) étant maintenu contre le cadre de fixation extérieur (52, 53), ledit au moins un élément support (48, 49) comportant un deuxième cadre de fixation (54, 55) intérieur et le cadre de fixation intérieur (54, 55) étant agencé à l'intérieur du premier cadre de fixation extérieur (52, 53) et étant relié à celui-ci par l'intermédiaire d'éléments de liaison (56, 57), **caractérisée en ce que** le filtre stérile (50) est agencé entre lesdits deux éléments support et **en ce que** les éléments de liaison (56, 57) des deux éléments support (48, 49), assemblés l'un à l'autre, sont agencés en étant tournés l'un par rapport à l'autre autour d'un axe de rotation (38) orienté parallèlement à une direction normale par rapport à un plan défini par le filtre stérile (50).

2. Unité de filtrage selon la revendication 1, **caractérisée en ce que** ladite au moins une zone de préhension (72) est agencée sur au moins un élément support (49).

3. Unité de filtrage selon la revendication 1 ou 2, **caractérisée en ce que** ladite au moins une zone de préhension (72) comporte un évidement (76, 77) agencé sur au moins un élément support (49).

4. Unité de filtrage selon l'une des revendications précédentes, **caractérisée en ce qu'**il est prévu plusieurs zones de préhension (72) agencées sur au moins un élément support (49).

5. Unité de filtrage selon l'une des revendications précédentes, **caractérisée en ce que** le filtre stérile (50) est maintenu serré entre les éléments support (48, 49).

6. Unité de filtrage selon l'une des revendications précédentes, **caractérisée en ce que** le filtre stérile (50) est assemblé de manière inamovible, en particulier par collage ou soudage, à au moins un élément support (48, 49).

7. Unité de filtrage selon la revendication 5 ou 6, **caractérisée en ce que** les deux éléments support (48, 49) sont assemblés de manière inamovible l'un à l'autre, en particulier par collage ou soudage.

8. Unité de filtrage selon l'une des revendications précédentes, **caractérisée en ce que** le cadre de fixation extérieur (52, 53) et/ou le cadre de fixation intérieur (54, 55) sont réalisés avec une forme circulaire.

9. Unité de filtrage selon l'une des revendications précédentes, **caractérisée en ce que** les éléments de liaison comportent des barrettes (56, 57) radiales, orientées en s'éloignant du centre des cadres de fixation (52, 53 ; 54, 55) extérieur et intérieur.

10. Unité de filtrage selon l'une des revendications précédentes, **caractérisée en ce que** des éléments de liaison (156, 157) des deux éléments support (48, 49) reliés l'un à l'autre sont agencés en se recouvrant dans une direction normale (138) par rapport à un plan défini par le filtre stérile (150).

11. Unité de filtrage selon l'une des revendications précédentes, **caractérisée en ce que** les éléments de liaison (56, 57), agencés en étant tournés l'un par rapport à l'autre, des deux éléments support (48, 49), reliés l'un à l'autre, sont décalés l'un par rapport à l'autre selon un angle de rotation (84), qui résulte du quotient entre 360° et le double du nombre des éléments de liaison (56, 57).

12. Unité de filtrage selon l'une des revendications précédentes, **caractérisée en ce que** ladite au moins une zone de préhension (72) est agencée sur l'un des cadres de fixation (52, 53 ; 54, 55) extérieur et/ou intérieur.

13. Unité de filtrage selon l'une des revendications précédentes, **caractérisée en ce que** le cadre de fixation extérieur (52, 53) et/ou le cadre de fixation intérieur (54, 55) d'un élément de liaison ont une largeur dans le sens radial et **en ce que** ladite au moins une zone de préhension (72) a une largeur dans le sens radial qui est au moins par zones supérieure à la largeur de parties du cadre de fixation extérieur (52, 53) et/ou du cadre de fixation intérieur (54, 55), adjacentes à la zone de préhension (72).

14. Unité de filtrage selon l'une des revendications précédentes, **caractérisée en ce que** ladite au moins une zone de préhension (72) comporte un élément de retenue pour les doigts (80, 81).

15. Unité de filtrage selon la revendication 14, **caractérisée en ce que** l'élément de retenue pour les doigts comporte un bord (80, 81) entourant la surface de préhension (78, 79) de la zone de préhension (72).

16. Unité de filtrage selon l'une des revendications précédentes, **caractérisée en ce que** la surface de préhension (78, 79) est structurée ou rugueuse.

17. Conteneur stérile, en particulier destiné à recevoir et à conserver de manière stérile des ustensiles ou matériels chirurgicaux, comportant une enceinte formée par un fond du conteneur et des parois du conteneur, un couvercle pour fermer l'enceinte et une ouverture d'échange de gaz, pouvant être fermée par une unité de filtrage, **caractérisé en ce que** l'unité de filtrage est une unité de filtrage (34 ; 134) selon l'une des revendications précédentes.
